# EUROPEAN PATENT APPLICATION

(11) **EP 4 173 713 A1**
(43) Date of publication of application: **03.05.2023**
(21) Application number: 21306500.6
(22) Date of filing: 27.10.2021
(51) Int. Cl.: B01L 3/00, A61B 10/00, G01N 1/02

(54) **DEVICE FOR COLLECTING BIOLOGICAL SAMPLES**

(71) Applicant: BIC Violex Single Member S.A., 14569 Anoixi (GR); Société BIC, 92110 Clichy (FR)
(72) Inventor: Katsikas, Georgios, 145 69 Anoixi (GR); Kopelas, Panagiotis, 145 69 Anoixi (GR); Saltas, Efthymios, 145 69 Anoixi (GR); Michenaud, Etienne, 92110 Clichy (FR)
(74) Representative: Peterreins Schley

(57) **Abstract**

The present disclosure relates to a device for collecting one or more biological samples comprising a housing and a collector element, wherein the collector element allows the passage of liquid, and wherein the collector element extends between a first end and a second end, the first end of the collector element protruding out of the housing for collecting the one or more biological samples, the second end of the collector element being arranged within the housing. The device further comprises a fixation element for engaging the second end of the collector element, wherein the fixation element has at least two parts which can be moved relative to one another for engaging the second end of the collector element, and wherein the housing comprises a connector element having an internal shape which is designed to maintain the parts of the fixation element in their engaged position.

## Description

### Background of the present disclosure

The present disclosure relates to device for collecting one or more biological samples, which may be subsequently used for molecular biology processing techniques such as nucleic acid amplification and/or detection.

Polymerase Chain Reaction (PCR) is a known method of amplifying nucleic acids, employed to test for the presence of specific nucleic acids (DNA or RNA) in a biological sample. It is used among other purposes as a diagnostic method to identify markers for pathogens or diseases. Other methods of amplifying nucleic acid samples include isothermal amplification methods such as Recombinase Polymerase Amplification (RPA) and Loop-Mediated Isothermal Amplification (LAMP).

Devices for collecting one or more biological samples - in particular for collecting saliva from the mouth of the consumer - are known from WO 2020/191232 A1, WO 2020/106345 A1, WO 2020/238035 A1 and EP 3 028 030 A1. Those devices comprise a wick as collector element. However, the wick is very soft and is difficult to maintain and fasten into the housing of the device.

The object of the present disclosure is to provide a device for collecting one or more biological samples having a collector element which can be mounted more easily on the device. In addition, the collector element should be securely mounted and maintained within the housing of the device to avoid an undesired removal.

### Summary of the present disclosure

The present disclosure is directed to a device for collecting one or more biological samples as defined in independent claim 1. The dependent claims depict embodiments of the present disclosure.

According to the present disclosure, a device for collecting one or more biological samples is provided which comprises a housing and a collector element, wherein the collector element is configured to allow the passage of liquid. It should be noted that the housing may comprise several separate housing parts. The collector element extends between a first end of the collector element protruding out of the housing for collecting the one or more biological samples, and a second end of the collector element being arranged within the housing. The first end may be arranged opposite to the first end.

According to the present disclosure, a fixation element is provided for engaging the second end of the collector element, wherein the fixation element has at least two parts which can be moved relative to one another for engaging the second end of the collector element, and wherein the housing comprises a connector element having an internal shape which is designed to maintain the parts of the fixation element in their engaged position. In particular, the fixation element may have at least one surface which is in contact with the collector element and which compresses the second end of the collector element. With these features, the collector element can securely be clamped between the parts of the fixation element and then mounted into the connector element.

In addition to this clamping actuation or alternatively, the fixation element may have at least one anchor element which extends at least partially into the collector element for maintaining the position of the collector element. The at least one anchor element may have the shape of a protrusion or a pin to secure the collector element.

The at least two parts of the fixation element may be connected by at least one hinge element such that the collector element can be embraced by placing the collector element into one part of the fixation element and by rotating the other part of the fixation element on the collector element. In examples, the two parts of the fixation element may be separate parts, wherein the collector element can be embraced by placing the collector element into one part of the fixation element and by arranging the other part of the fixation element on the collector element.

In embodiments, the fixation element may have radially extending ribs which are held by grooves which are formed within the connector element. These features ensure that the fixation element is securely held withing the connector element, and that the collector element remains its position within the connector element.

The connector element may be mounted on the main body of the housing on the side of the collector element. Alternatively, the connector element may be an integral part of the housing.

In all embodiments of the present disclosure, the following additional features may be provided.

The collector element may generally have an elongated shape extending from the first end to the second end of the collector element. In the following disclosure, the axis of the collector element is defined as being the central gravitational axis extending from the first end to the second end. The cross-sectional shape of the collector element (in a plane which is perpendicular to the axis of the collector element) may at least partially be circular, oval or rectangular, in examples with rounded corners. The section of the collector element at its first end may have a conical section. In addition or alternatively, the first end of the collector element may be pointed.

The purpose of the collector element is to collect and/or absorb one or more biological samples. In case of dry biological samples, the biological samples may stick on the outer surface of the first end of the collector element. In case of biological samples which are contained in a liquid, the biological samples may be absorbed within the collector element. Therefore, the collector element should allow the passage of liquid at least partially in order to take up absorb a greater amount of the biological sample(s). The material of the collector element may be porous or may have an internal channel structure, and has, in examples hydrophilic properties in order to absorb a large amount of liquid biological samples more quickly.

Suitable materials for the collector element include cotton-based materials, or a plastic based matrix such as PE (polyethylene), PP (polypropylene) or PVDF (polyvinylidene fluoride) or a combination thereof, which can be manufactured in a range of densities to retain biological samples of different viscosities. In examples, the collector element may be made of a combination of PE and PP, wherein the PP may be the core portion and the PE may be the outer coating, due to its particular properties on absorption of liquid via its capillary structure, and adsorption which is minimal with regards to nucleic acids. The collector element may comprise fibers (e.g pressed fibers) made of one or more of these materials. The material of the collector element may be porous or may have an internal channel structure, and has preferably hydrophilic properties in order to absorb a large amount of liquid biological samples more quickly. The porosity may be at least 50%, more specifically at least 70%, most specifically at least 80%. The density of the collector element may be between 0.1 to 0.25 g/cm³.

In addition, as will be explained below in more detail, the collector element may be configured to allow the passage of liquid from a container, in particular a buffer solution and/or a reagent solution, through the collector element towards its first end in order to drain the biological sample(s) out of the collector element. In particular, the collector element may allow the passage of liquid from its second end to its first end, or from the outer surface near the second end to the first end.

As already mentioned, the collector element may be soft and/or flexible such that the anchor element can extend at least partially into the collector element. In examples, the device may comprise an abutment surface or a stop for limiting the movement of the collector element into the housing.

The device may further comprise a container for storing liquid, in particular a buffer solution and/or a reagent solution therein. In addition, the housing may comprise or enclose a channel for guiding the liquid stored in the container to the collector element. The device may further comprise a release mechanism for opening a passage from the container to the channel so that liquid stored in the container can enter the channel from the container and reach the collector element. This release mechanism may, for example, be a valve or a cover sheet on one side of the container which can be penetrated by a needle by moving the container further into the housing.

Therefore, a liquid stored in the container can reach the collector element through the channel when the release mechanism is open, wherein at least part of the liquid can pass through the collector element by means of gravity acting on the liquid when the container is in a position vertically above the collector element. In examples, the volume of the container can be reduced after the release mechanism has opened the fluid passage from the container, for example by means of a movable plunger or by providing the container with flexible walls such that the liquid can be squeezed out of the container into the channel.

When the liquid from the container passes through the collector element, the biological sample can at least partially be washed out of the collector element for testing/analysis purposes.

The device may further comprise a cap element which can be fitted on the housing on the side on which the collector element is arranged. An element for indicating and/or measuring a reaction between a reagent and the at least one biological sample may be arranged within or on the cap element, in particular an element indicating the pH value of the mixture of the liquid from the container and of the at least one biological sample. In addition or alternatively, the cap element may comprises a reagent or an element comprising a reagent which is released to the mixture of liquid from the container and of the at least one biological sample. With these features, the device can be used not only for collecting biological samples, but also to analyze/test the samples on site (i.e. without the need of sending the device to a laboratory).

In examples, the cap element may comprise a window which is arranged such that a user can at least partially see the element for indicating and/or measuring a reaction between the reagent and the at least one biological sample.

### Short description of the figures

- Figure 1: shows a device for collecting one or more biological samples according to an embodiment of the present disclosure;
- Figures 2a to 2d: show the anchoring of a collector element according to a first embodiment of the present disclosure;
- Figure 3: shows a cross-sectional drawing of a device according to the present disclosure.

### Detailed description of the present disclosure

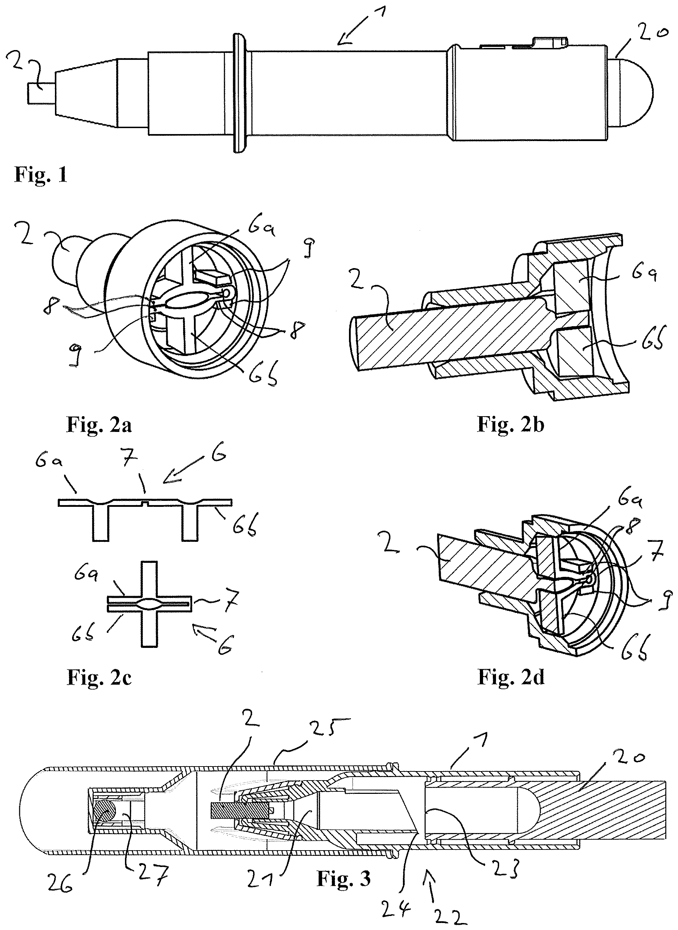

Figure 1 shows a device for collecting one or more biological samples according to an embodiment of the present disclosure. The device comprises a housing 1 and a collector element 2, wherein the collector element 2 allows the passage of liquid. As already mentioned, the housing may consist of several separate housing parts. The collector element 2 extends between a first end of the collector element protruding out of the housing 1 for collecting the one or more biological samples, and a second end of the collector element 2, opposite to the first end, which is arranged within the housing 1. Regarding the softness range, the shape, the absorption properties and the material of the collector element 2, it is referred to the above summary section of the present disclosure. The device may also comprise a container 20 for storing liquid, in particular a buffer solution and/or a reagent solution therein as will be explained in more detail in context with Figure 3.

Figures 2a to 2d show the anchoring of a collector element according to a first embodiment of the present disclosure. According to this embodiment, a fixation element 6 is provided for engaging the second end of the collector element 2, wherein the fixation element 6 has at least two parts 6a, 6b which can be moved relative to one another for engaging the second end of the collector element 2, and wherein the housing comprises a connector element 3 having an internal shape which is designed to maintain the parts of the fixation element 6 in their engaged position. The fixation element 6 may have at least one surface which is in contact with the collector element 2 and which compresses the second end of the collector element. With these features, the collector element 2 can be securely clamped between the parts of the fixation element 6 and then mounted into the connector element 3.

In addition to this clamping actuation or alternatively, the fixation element may have at least one anchor element (not shown in Figures 2a to 2d) which extends at least partially into the collector element for maintaining the position of the collector element. The at least one anchor element may have the shape of a protrusion or a pin to secure the collector element.

The at least two parts of the fixation element may be connected by at least one hinge element 7 such that the collector element can embraced by placing the collector element into one part of the fixation element and by rotating the other part of the fixation element on the collector element. In examples, the two parts 6a, 6b of the fixation element 6 may be separate parts, wherein the collector element can be embraced by placing the collector element into one part of the fixation element and by arranging the other part of the fixation element on the collector element.

In all embodiments, the fixation element may have radially extending ribs 8 on two opposite sides which are held by grooves 9 which are formed within the connector element. These features ensure that the fixation element is securely held withing the connector element, and that the collector element remains its position within the connector element.

The connector element 3 may be mounted on the main body of the housing on the side of the collector element. Alternatively, the connector element may be an integral part of the housing.

In all embodiments of the present disclosure, the device may comprise an abutment surface or a stop for limiting the movement of the collector element 2 into the housing.

In all embodiments of the present disclosure, the collector element may have a softness as described above in the summary section such that the anchor element can extend at least partially into the collector element.

Suitable materials for the collector element include cotton-based materials, or specifically a plastic based matrix such as PE, PP or PVDF which can be manufactured in a range of densities to absorb and retain biological samples of different viscosities. The material of the collector element may be porous or may have an internal channel structure, and has, in examples, hydrophilic properties in order to absorb a large amount of liquid biological samples more quickly. The porosity should be at least 50%, more specifically at least 70%, most specifically at least 80%.

Figure 3 shows a cross-sectional drawing of a device according to the present disclosure in which the anchoring of the collector element 2 may be designed according to one of the embodiments described in this disclosure (wherein Figure 3 shows an alternative anchoring of the collector element). The device may comprise a container 20 for storing liquid, in particular a buffer solution. In addition, the housing may comprise or enclose a channel 21 for guiding the liquid stored in the container 20 to the collector element 2. The device may further comprise a release mechanism 22 for opening a passage from the container 20 to the channel 21 so that liquid stored in the container can enter the channel 21 from the container 20 and reach the collector element 2. This release mechanism may, for example, be a valve or a cover sheet 23 on one side of the container which can be penetrated by a needle or by a penetrating device 24 by moving the container further into the housing.

Therefore, liquid stored in the container can reach the collector element 2 through the channel 21 when the release mechanism 22 is open, wherein at least part of the liquid can pass through the collector element 2 by means of gravity acting on the liquid when the container is in a position vertically above the collector element. Alternatively, the volume of the container 20 be designed to be reduced after the release mechanism has opened the fluid passage from the container, for example by means of a movable plunger or by providing the container 20 with flexible walls such that the liquid can be squeezed out of the container into the channel.

When the liquid from the container 20 passes through the collector element 2, the biological sample can at least partially be washed out of the collector element 2 for testing/analysis purposes.

The device may further comprise a cap element 25 which can be fitted on the housing on the side on which the collector element is arranged. The cap element may comprise a reagent 26 or an element comprising a reagent which is released to the mixture of liquid from the container and of the at least one biological sample. This element comprising a reagent may have the shape of a pellet. In addition, an element 27 for indicating and/or measuring a reaction between a reagent 26 and the at least one biological sample may be arranged in a reaction chamber of the cap element, in particular an element indicating the pH value of the mixture of the liquid from the container and of the at least one biological sample. With these features, the device can be used not only for collecting biological samples, but also to analyze/test the samples on site (i.e. without the need of sending the device to a laboratory).

The cap element 25 may comprise a window (not shown in the Figures) which is arranged such that a user can at least partially see the element for indicating and/or measuring a reaction between the reagent and the at least one biological sample. Alternatively, the cap element may be manufactured of a transparent material.

According to this disclosure, various amplification methods may be used, for example a recombinase polymerase amplification (RPA) which is a low temperature DNA and/or RNA amplification technique, or a ligase chain reaction (LCR).

The buffer liquid stored in the container 20 may contain purified water, milli-q water and buffer (e.g. TRIS-C1 / TE buffer), for example a Tris-HCl buffer. When LCR method is applied, buffer also contains PEG as crowding agent.

The reagents located in the cap may include a group of materials, such as primers, probes additionally to the enzymes (ligase or polymerase) and nucleotides. Generally, the reagents located in the cap are dependent on the used amplification method. A suitable reagent composition for RPA may contain enzymes (in particular enzymes for polymerase and/or enzymes for reverse transcriptase), proteins, primers, probes, oligonucleotides and reaction components necessary for the RPA reaction including reaction buffer, polyethylene glycol, pyrophosphatase, a regeneration system such as kinase/phosphocreatine system, or a mixture thereof. A suitable reagent composition for LCR may contain enzymes for DNA ligase and/or DNA probes.

Possible materials for the collector element have already been described. The collector element may additionally be treated. For example, reagents or active agents may be covalently bound to the collector element by chemical linkages through functionalisation (e.g. -OH groups), or they may be dried into the material of the collector element and become active on hydration with the sample. Alternatively or in addition, the collector element may be treated to provide ways of lysing virus, in particular a treatment to form a negatively charged surface (so as to retain the positively charged ions of the sample) or a treatment with anionic detergent. Possible active agents that render the collector element functionalized may lyse bacteria or viruses. These active agents may be quaternary ammonium compounds (QAC) and/or biocidal agents. Examples of these active agents are: Citral, Eucalyptus oil, Tea tree oil, Chlorhexidine, triterpenoid saponin, Polyphylla saponin I, Povidone-iodine, Cetyl pyridinium chloride (CPC), Benzalkonium chloride (BAC), Dequoalinium chloride.

Although the device of the present disclosure is defined in the following claims, it should be understood that the present disclosure can alternatively and/or in addition be defined according to the following embodiments:
1. A device for collecting one or more biological samples comprising a housing and a collector element,
   wherein the collector element allows the passage of liquid, and
   wherein the collector element extends between a first end and a second end,
      the first end of the collector element protruding out of the housing for collecting
      the one or more biological samples,
      the second end of the collector element being arranged within the housing,
   wherein the devide further comprises a fixation element for engaging the second end of the collector element, wherein the fixation element has at least two parts which can be moved relative to one another for engaging the second end of the collector element, and wherein the housing comprises a connector element having an internal shape which is designed to maintain the parts of the fixation element in their engaged position.
2. Device according to embodiment 1, wherein the fixation element has at least one surface which is in contact with the collector element and which compresses the second end of the collector element.
3. Device according to embodiment 1 or 2, wherein the fixation element has at least one anchor element which extends at least partially into the collector element for maintaining the position of the collector element.
4. Device according to one of the preceding embodiments, wherein the at least two parts of the fixation element are connected by means of at least one hinge element such that the collector element can embraced by placing the collector element into one part of the fixation element and by rotating the other part of the fixation element on the collector element.
5. Device according to one of the preceding embodiments, wherein the two parts of the fixation element are separate parts, wherein the collector element can be embraced by placing the collector element into one part of the fixation element and by arranging the other part of the fixation element on the collector element.
6. Device according to one of the preceding embodiments, wherein the fixation element has at least two radially extending ribs which are held by grooves which are formed within the connector element.
7. Device according to one of the preceding embodiments, wherein the connector element can be mounted on the main body of the housing on the side of the collector element.
8. Device according to one of the preceding embodiments, wherein the collector element is soft and/or flexible such that the anchor element can extend at least partially into the collector element.
9. Device according to one of the preceding embodiments, further comprising an abutment surface or a stop for limiting the movement of the collector element into the housing.
10. Device according to one of the preceding embodiments, further comprising a container for storing liquid, in particular a buffer solution and/or a reagent solution therein.
11. Device according to one of the preceding embodiments, wherein the housing comprises or encloses a channel for guiding the liquid stored in the container to the collector element.
12. Device according to one of the preceding embodiments, further comprising a valve and/or release mechanism for opening a passage from the container to the channel so that liquid stored in the container can enter the channel from the container and reach the collector element.
13. Device according to one of the preceding embodiments, wherein liquid stored in the container can reach the collector element through the channel when the valve and/or release mechanism is open, wherein at least part of the liquid can pass through the collector element by means of gravity acting on the liquid when the container is in a position vertically above the collector element.
14. Device according to one of the preceding embodiments, wherein the volume of the container can be reduced to squeeze liquid from the container into the channel.
15. Device according to one of the preceding embodiments, wherein the volume of the container can be reduced such that liquid is released from the container into the channel and passes through the collector element.
16. Device according to one of the preceding embodiments, further comprising a cap element which can be fitted on the housing on the side on which the collector element is arranged.
17. Device according to one of the preceding embodiments, characterized in that an element for indicating and/or measuring a reaction between a reagent and the at least one biological sample is arranged within or on the cap element, in particular an element indicating the pH value of the mixture of the liquid from the container and of the at least one biological sample.
18. Device according to one of the preceding embodiments, characterized in that the cap element comprises a reagent or an element comprising a reagent which is released to the mixture of liquid from the container and of the at least one biological sample.
19. Device according to one of embodiments 16 to 18, characterized in that the cap element preferably comprises a window which is arranged such that a user can at least partially see the element for indicating and/or measuring a reaction between the reagent and the at least one biological sample.

## Claims

1. A device for collecting one or more biological samples comprising a housing and a collector element,
wherein the collector element allows the passage of liquid, and
wherein the collector element extends between a first ends and a second end,
the first end of the collector element protruding out of the housing for collecting
the one or more biological samples,
the second end of the collector element being arranged within the housing,
wherein the device further comprises a fixation element for engaging the second end of the collector element, wherein the fixation element has at least two parts which can be moved relative to one another for engaging the second end of the collector element, and
wherein the housing comprises a connector element having an internal shape which is designed to maintain the parts of the fixation element in their engaged position.

2. Device according to claim 1, wherein the fixation element has at least one surface which is in contact with the collector element and which compresses the second end of the collector element.

3. Device according to claim 1 or 2, wherein fixation element has at least one anchor element which extends at least partially into the collector element for maintaining the position of the collector element.

4. Device according to one of the preceding claims, wherein the at least two parts of the fixation element are connected by means of at least one hinge element such that the collector element can embraced by placing the collector element into one part of the fixation element and by rotating the other part of the fixation element on the collector element.

5. Device according to one of the preceding claims, wherein the two parts of the fixation element are separate parts, wherein the collector element can be embraced by placing the collector element into one part of the fixation element and by arranging the other part of the fixation element on the collector element.

6. Device according to one of the preceding claims, wherein the fixation element has at least two radially extending ribs which are held by grooves which are formed within the connector element.

7. Device according to one of the preceding claims, wherein the connector element can be mounted on the main body of the housing on the side of the collector element.

8. Device according to one of the preceding claims, further comprising an abutment surface or a stop for limiting the movement of the collector element into the housing.

9. Device according to one of the preceding claims, further comprising a container for storing liquid, in particular a buffer solution and/or a reagent solution therein.

10. Device according to one of the preceding claims, wherein the housing comprises or encloses a channel for guiding the liquid stored in the container to the collector element.

11. Device according to one of the preceding claims, further comprising a valve and/or release mechanism for opening a passage from the container to the channel so that liquid stored in the container can enter the channel from the container and reach the collector element.

12. Device according to one of the preceding claims, wherein liquid stored in the container can reach the collector element through the channel when the valve and/or release mechanism is open, wherein at least part of the liquid can pass through the collector element by means of gravity acting on the liquid when the container is in a position vertically above the collector element.

13. Device according to one of the preceding claims, further comprising a cap element which can be fitted on the housing on the side on which the collector element is arranged.

14. Device according to one of the preceding claims, wherein an element for indicating and/or measuring a reaction between a reagent and the at least one biological sample is arranged within or on the cap element, in particular an element indicating the pH value of the mixture of the liquid from the container and of the at least one biological sample.

15. Device according to one of the preceding claims, wherein the cap element comprises a reagent or an element comprising a reagent which is released to the mixture of liquid from the container and of the at least one biological sample.
